# EUROPÄISCHE PATENTSCHRIFT

(11) **EP 1 099 683 B1**
(45) Veröffentlichungstag und Bekanntmachung des Hinweises auf die Patenterteilung: **04.06.2003**
(21) Anmeldenummer: 00123374.1
(22) Anmeldetag: 31.10.2000
(51) Int. Cl.: C07C 201/00, C07C 207/02, C07C 207/04

(54) **Verfahren zur Herstellung von Nitrosobenzolen**
Process for the preparation of nitrosobenzenes
Procédé pour la préparation de nitrosobenzènes

(30) Priorität: 12.11.1999 DE 19954396
(43) Veröffentlichungstag der Anmeldung: 16.05.2001
(73) Patentinhaber: Bayer Aktiengesellschaft, 51368 Leverkusen (DE)
(72) Erfinder: Giera, Henry, Dr., 86862 Grosskitzighofen (DE); Lange, Walter, Dr., 51519 Odenthal (DE); Meiers, Michaela, Dr., 67346 Speyer (DE); Pires, Raul, Dr., 51373 Leverkusen (DE)

(56) Entgegenhaltungen:
- DATABASE WPI Section Ch, Week 199401 Derwent Publications Ltd., London, GB; Class B05, AN 1994-005122 XP002161864 & SU 1 781 207 A (SIBE TECHN INST), 15. Dezember 1992 (1992-12-15)
- TOLLARI, STEFANO ET AL: "Catalytic oxidation of primary aromatic amines to the corresponding nitroso compounds by H2O2 and [Mo(O)(O2)2(H2O)(hmpa)] (hmpa = hexamethylphosphoric triamide)" J. CHEM. SOC., CHEM. COMMUN. (1993), (19), 1510-11 , XP002161859
- PORTA, F. ET AL: "Catalytic synthesis of C-nitroso compounds by cis-Mo(O)2(acac)2" J. MOL. CATAL. A: CHEM. (2000), 157(1-2), 123-129 , XP000982547

## Beschreibung

Die Erfindung betrifft ein Verfahren zur Herstellung von Nitrosobenzolen aus aromatischen Aminen durch Oxidation mit Wasserstoffperoxid in Gegenwart eines Katalysators und eines geeigneten inerten organischen Lösungsmittels.

Die Herstellung von Nitrosobenzol, bei dem es sich um ein wichtiges Zwischenprodukt handelt, ist bekannt und kann nach verschiedenen Methoden durchgeführt werden. Es kann zur Synthese von Alterungsschutzmitteln und Stabilisatoren in der Gummi- und Polymerindustrie, insbesondere zur Herstellung von 4-Aminodiphenylamin (4-ADPA) eingesetzt werden.

Eine Methode zur Herstellung von Nitrosobenzolen ist die Oxidation von entsprechenden aromatischen Aminen mit geeigneten Oxidationsmitteln, wie Wasserstoffperoxid, in Anwesenheit eines Katalysators sowie in Gegenwart eines geeigneten Lösungsmittels.

Die Herstellung von Nitrosobenzolen aus aromatischen Aminen durch katalytische Oxidation mit Wasserstoffperoxid ist beispielsweise beschrieben von S. Sakaue, T. Tsubakino, Y. Nishiyama, Y. Ishii. *Org. Chem.* **1993**, 58, 3633; S. Tollari, M. Cuscela, F. Porta, *J. chem. Soc., Chem. Commun.* **1993**, 1510; sowie in RU-A 1680689; RU-A 2042661; RU-A 2044724; RU-A 2090542; und von E.B. Mel'nikov, G.A. Suboch, E. Yu. Belyaev, *Russ. J. Org. Chem.* **1995,** 31, 160-1642; und Z. Zhu, J.H. Espensen, *J Org. Chem.* **1995**, 60, 1326-1332.

Nachteile bei den angegebenen Verfahren sind z.B. der Einsatz großtechnisch nicht praktikabler Mengen an risikobelastetem Lösungsmittel, wie Chloroform, um gute Ausbeuten zu erzielen, die Verwendung von toxischen Verbindungen wie Hexamethylphosphorsäuretriamid bei der Katalysatorherstellung, die Verwendung von zusätzlichen Co-Katalysatoren und der Einsatz größerer Katalysatormengen sowie die Verwendung von teuren Katalysatoren. Durch die gegebenen Nachteile würde sich ein großtechnisch betriebenes Verfahren unwirtschaftlich gestalten verbunden mit zum Teil umfangreichen Sicherheitsmaßnahmen.

Es wurde nun gefunden, daß man Nitrosobenzole aus aromatischen Aminen durch katalytische Oxidation mit Wasserstoffperoxid in technisch einfacher Weise und unter Vermeidung der oben geschilderten Nachteile herstellen kann, wenn man die Oxidation in Gegenwart von Natriummolybdat als Katalysator durchführt.

Gegenstand der vorliegenden Erfindung ist daher ein Verfahren zur Herstellung von Nitrosobenzolen der allgemeinen Formel 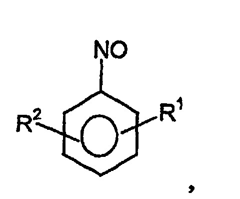 worin
- R¹ und R²: gleich oder verschieden sind und für Wasserstoff, C₁-C₄-Alkyl oder C₁-C₄-Alkoxy stehen,
durch Oxidation entsprechender aromatischer Amine in Anwesenheit eines inerten organischen Lösungsmittels oder Lösungsmittelgemisches, das dadurch gekennzeichnet ist, dass man die Oxidation mit Wasserstoffperoxid in Gegenwart von Natriummolybdat-Dihydrat bei Temperaturen von 5 bis 25°C bei einem Molverhältnis von aromatischen Aminen zu Wasserstoffperoxid zu Natriummolybdat-Dihydrat von 1:2,0 bis 5,0:0,001 bis 0,05 durchführt. Bevorzugt eingesetzt werden aromatische Amine, wie z.B. Anilin, o-, m- und p-Toluidin, o-, m-, p-Anisidin, ganz besonders bevorzugt Anilin.

Bevorzugt werden Nitrosobenzole der obigen Formel hergestellt in denen R¹ und R² für Wasserstoff, Methyl- oder Methoxy-Gruppen stehen. Ganz besonders bevorzugt ist die Herstellung von Nitrosobenzol (d.h. R¹ und R² = Wasserstoff).

Das erfindungsgemäße Verfahren wird bevorzugt bei Temperaturen von 15 bis 25°C durchgeführt.

Das erfindungsgemäß eingesetzte Wasserstoffperoxid wird üblicherweise in wässriger Form in einer Konzentration von 15 bis 80, bevorzugt 30 bis 50, Gew.-% Wasserstoffperoxid eingesetzt.

Als inerte organische Lösungsmittel kommen insbesondere solche in Betracht, die mit Wasser praktisch nicht mischbar sind und eine zweite (organische) Phase im Reaktionsgemisch auszubilden vermögen.

Insbesondere sind als einzusetzende organische Lösungsmittel zu nennen: Cyclohexan, Petrolether, Toluol und/oder Dichlormethan, insbesondere Cyclohexan, Petrolether und/oder Toluol.

Wie erwähnt können die inerten organischen Lösungsmittel, die mit Wasser praktisch nicht mischbar sind, auch in Mischungen untereinander eingesetzt werden. Das günstigste Mischungsverhältnis kann leicht durch entsprechende Vorversuche bestimmt werden, ebenso die Menge an inerten organischen Lösungsmitteln.

Bevorzugt wird das erfindungsgemäße Verfahren bei einem Molverhältnis von aromatischen Aminen zu Wasserstoffperoxid zu Natriummolybdat-Dihydrat von 1:2,5 bis 4,5:0,005 bis 0,02 durchgeführt.

Nach Beendigung des erfindungsgemäßen Verfahrens kann das erhaltene Nitrosobenzol in üblicher Weise aus der Reaktionsmischung erhalten werden, durch zum Beispiel Abtrennen der organischen Phase, Waschen mit einer NaCl-Lösung und Entfernung des eingesetzten Lösungsmittels.

Die Nitrosobenzole werden in Ausbeuten von 64 bis 92 % der Theorie und in Reinheiten von ≥95 % erhalten.

### Beispiele

### Allgemeine Arbeitsvorschrift:

Der Katalysator wird vorgelegt, 1 mmol (93,13 mg) Anilin gelöst in 2,5 ml Lösungsmittel werden zugesetzt und mit Wasserstoffperoxid versetzt. Nach 4 h wird die Reaktion beendet und die Ausbeute wird mit einer GC-Methode bestimmt gemäß:
1.) Vogel's, Textbook of Quantitative Chemical Analysis, 5^{th} Edition, Longwan Scientific & Technical, S. 247
2.) H. Hulpke, H. Hartkamp, G. Tölg (Hrsg.), Analytische Chemie für die Praxis, K. Beyermann, Organische Spurenanalyse, G. Thieme Verlag, Stuttgart, New York, 1982, S. 37-41.

**Tabelle:**

| Zusammenfassung der Beispiele | | | | |
|---|---|---|---|---|
| Beispiel Nr. | Lösungsmittel | Kat.-Menge (Äq.) | H₂O₂-Menge (Äq.) | Ausbeute (%) |
| 1 | Cyclohexan | 0,02 | 3,0 | 87 |
| 2 | Cyclohexan | 0,02 | 3,4 | 90 |
| 3 | Cyclohexan | 0,01 | 3,0 | 84 |
| 4 | Cyclohexan | 0,01 | 3,4 | 88 |
| 5 | Petroether | 0,02 | 3,0 | 90 |
| 6 | Petrolether | 0,02 | 3,4 | 92 |
| 7 | Petrolether | 0,01 | 3,0 | 89 |
| 8 | Petrolether | 0,01 | 3,4 | 88 |
| 9 | Toluol | 0,02 | 3,0 | 67 |
| 10 | Toluol | 0,02 | 3,4 | 69 |
| 11 | Toluol | 0,01 | 3,0 | 64 |

## Patentansprüche

1. Verfahren zur Herstellung von Nitrosobenzolen der allgemeinen Formel 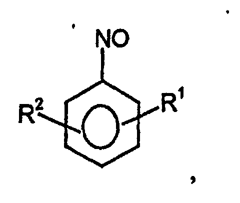 worin
R¹ und R² gleich oder verschieden sind und für Wasserstoff, C₁-C₄-Alkyl oder C₁-C₄-Alkoxy stehen,
durch Oxidation entsprechender aromatischer Amine in Anwesenheit eines inerten organischen Lösungsmittels oder Lösungsmittelgemisches, das **dadurch gekennzeichnet ist, dass** man die Oxidation mit Wasserstoffperoxid in Gegenwart von Natriummolybdat-Dihydrat bei Temperaturen von 5 bis 25°C bei einem Molverhältnis von aromtischen Aminen zu Wasserstoffperoxid zu Natriummolybdat-Dihydrat von 1:2,0 bis 5,0:0,001 bis 0,05 durchführt.

2. Verfahren zur Herstellung von Nitrosobenzolen gemäß Anspruch 1, **dadurch gekennzeichnet, dass** man bei einem Molverhältnis von aromatischen Aminen zu Wasserstoffperoxid zu Natriummolybdat-Dihydrat von 1:2,5 bis 4,5:0,005 bis 0,02 arbeitet.

3. Verfahren zur Herstellung von Nitrosobenzolen gemäß Anspruch 1, **dadurch gekennzeichnet, dass** man als organische Lösungsmittel Cyclohexan, Petrolether, Toluol und/oder Dichlormethan einsetzt.

## Claims

1. Process for the preparation of nitrosobenzenes of the general formula 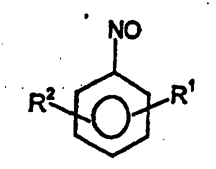 wherein
R¹ and R² are identical or different and represent hydrogen, C₁-C₄-alkyl or C₁-C₄-alkoxy,
by oxidation of corresponding aromatic amines in the presence of an inert organic solvent or solvent mixture, which is **characterized in that** the oxidation is carried out with hydrogen peroxide in the presence of sodium molybdate dihydrate at temperatures of 5 to 25°C at a molar ratio of aromatic amines to hydrogen peroxide to sodium molybdate dihydrate of 1:2.0 to 5.0:0.001 to 0.05.

2. Process for the preparation of nitrosobenzenes according to claim 1, **characterized in that** it is carried out at a molar ratio of aromatic amines to hydrogen peroxide to sodium molybdate dihydrate of 1:2.5 to 4.5:0.005 to 0.02.

3. Process for the preparation of nitrosobenzenes according to claim 1, **characterized in that** cyclohexane, petroleum ether, toluene and/or methylene chloride are employed as organic solvents.

## Revendications

1. Procédé de préparation de nitrosobenzènes de la formule générale : 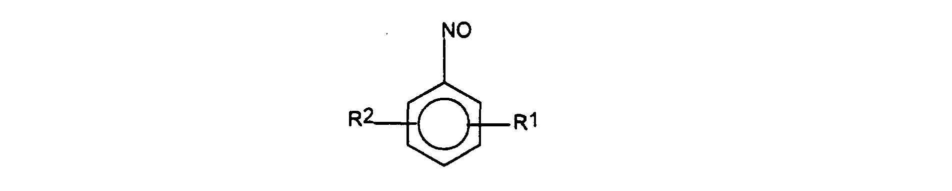 où R¹ et R² sont identiques ou différents et représentent l'atome d'hydrogène, un radical alcoyle en C₁-C₄ ou alcoxy en C₁-C₄,
par oxydation de l'amine aromatique correspondante en présence d'un solvant organique inerte ou d'un mélange de solvants, qui est **caractérisé en ce que** l'on réalise l'oxydation avec le peroxyde d'hydrogène en présence de molybdate de sodium dihydraté à des températures allant de 5 à 25°C, à un rapport molaire des amines aromatiques au peroxyde d'hydrogène au molybdate de sodium dihydraté de 1:2,0 à 5,0:0,001 à 0,05.

2. Procédé de préparation de nitrosobenzènes selon la revendication 1, **caractérisé en ce que** l'on travaille à un rapport molaire des amines aromatiques au peroxyde d'hydrogène au molybdate de sodium dihydraté allant de 1:2,5 à 4,5:0,005 à 0,02.

3. Procédé de préparation de nitrosobenzènes selon la revendication 1, **caractérisé en ce que** l'on met en oeuvre comme solvant organique, le cyclohexane, l'éther de pétrole, le toluène et/ou le dichlorométhane.
